(19)

Europäisches
Patentamt
European
Patent Office
Office européen
des brevets

(11) **EP 2 072 529 A1**

(12) **EUROPEAN PATENT APPLICATION**

(43) Date of publication:
**24.06.2009 Bulletin 2009/26**

(51) Int Cl.:
***C07K 14/74*** *(2006.01)* ***G01N 33/52*** *(2006.01)*
***C11B 9/00*** *(2006.01)*

(21) Application number: **07025035.2**

(22) Date of filing: **21.12.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **basisnote AG**
**3011 Bern (CH)**

(72) Inventors:
• **Hämmerli, August**
**3007 Bern (CH)**

• **Kaegi, Manuel**
**3007 Bern (CH)**
• **Senn, Dominic**
**3011 Bern (CH)**

(74) Representative: **Kalhammer, Georg et al**
**Lederer & Keller**
**Patentanwälte**
**Prinzregentenstrasse 16**
**80538 München (DE)**

(54) **MHC rapid assay used for the customization of odours**

(57) The present invention provides a user friendly test kit for determining an individual's Major Histocompatibilty profile and using that profile for the customization of odours.

EP 2 072 529 A1

SEQUENCE LISTING

<110> Basisnote AG

<120> MHC rapid assay used for the customization of odours

<130> custom odours

<160> 22

<170> PatentIn version 3.3

<210> 1
<211> 10
<212> PRT
<213> artificial

<220>
<223> Ligand of HLA-A*01 (A1)

<400> 1

Tyr Thr Asp Leu Leu Arg Leu Phe Glu Tyr
1               5               10


<210> 2
<211> 12
<212> PRT
<213> artificial

<220>
<223> Ligand of HLA-A*02 (A2)

<400> 2

Ser Leu Leu Thr Ser Ser Lys Gly Gln Leu Gln Lys
1               5               10


<210> 3
<211> 10
<212> PRT
<213> artificial

<220>
<223> Ligand of HLA-A*24 (A24)

<400> 3

Glu Tyr Pro Asp Arg Ile Met Asn Thr Phe
1               5               10


<210> 4
<211> 11
<212> PRT
<213> artificial

<220>
<223> Ligand of HLA-A*26 (A26)

<400> 4

Glu Thr Phe Tyr Val Asp Gly Ala Ala Asn Arg
1               5               10


<210> 5

<211> 12
<212> PRT
<213> artificial

<220>
<223> Ligand of HLA-B*07 (B7)

<400> 5

Val Gly Gly Leu Lys Asn Trp Leu Val His Arg Leu
1               5                   10


<210> 6
<211> 9
<212> PRT
<213> artificial

<220>
<223> Ligand of HLA-B*08 (B8)

<400> 6

Met Pro His Glu Lys His Tyr Thr Leu
1               5


<210> 7
<211> 9
<212> PRT
<213> artificial

<220>
<223> Ligand of HLA-B*35 (B35)

<400> 7

Trp Ala Ser Arg Glu Leu Glu Arg Phe
1               5


<210> 8
<211> 10
<212> PRT
<213> artificial

<220>
<223> Ligand of HLA-B*40 (B40)

<400> 8

Arg Glu Ile Ala Gln Asp Phe Lys Thr Asp
1               5                   10


<210> 9
<211> 9
<212> PRT
<213> artificial

<220>
<223> Ligand of HLA-B*44 (B44)

<400> 9

Asp Glu Tyr Ile Tyr Arg His Phe Phe
1               5

```
<210>    10
<211>    10
<212>    PRT
<213>    artificial

<220>
<223>    Ligand of HLA-B*51 (B51)

<400>    10

Asp Thr Pro Leu Ile Pro Leu Thr Ile Phe
1               5                   10


<210>    11
<211>    9
<212>    PRT
<213>    artificial

<220>
<223>    Ligand of HLA-Cw*03 (Cw3)

<400>    11

Ser Ala Tyr Gly Glu Pro Arg Lys Leu
1               5


<210>    12
<211>    10
<212>    PRT
<213>    artificial

<220>
<223>    Ligand of HLA-DPB1*0201

<400>    12

Glu Ile Val Asp Leu Met Cys His Ala Thr
1               5                   10


<210>    13
<211>    16
<212>    PRT
<213>    artificial

<220>
<223>    Ligand of HLA-DPB1*0401

<400>    13

Ile Ala Phe Asn Ser Gly Met Glu Pro Gly Val Val Ala Glu Lys Val
1               5                   10                  15


<210>    14
<211>    15
<212>    PRT
<213>    artificial

<220>
<223>    Ligand of HLA-DQA1*0201

<400>    14

Gln Pro Gln Gln Pro Gln Gln Ser Phe Pro Glu Gln Glu Arg Pro
1               5                   10                  15
```

```
<210> 15
<211> 15
<212> PRT
<213> artificial

<220>
<223> Ligand of HLA-DQA1*0301

<400> 15
```

His Lys Leu Gln Asp Ala Ser Ala Glu Val Glu Arg Leu Arg Arg
1               5                   10                  15

```
<210> 16
<211> 16
<212> PRT
<213> artificial

<220>
<223> Ligand of HLA-DRB1*03 (DR3)

<400> 16
```

Ile Pro Glu Asn Leu Phe Leu Lys Ser Asp Gly Arg Val Lys Tyr Thr
1               5                   10                  15

```
<210> 17
<211> 18
<212> PRT
<213> artificial

<220>
<223> Ligand of HLA-DRB1*0301 (DR17)

<400> 17
```

Ile Ser Asn Gln Leu Thr Leu Asp Ser Asn Thr Lys Tyr Phe His Lys
1               5                   10                  15

Leu Asn

```
<210> 18
<211> 23
<212> PRT
<213> artificial

<220>
<223> Ligand of HLA-DRB1*0701 (DR7)

<400> 18
```

Lys Val Asp Leu Thr Phe Ser Lys Gln His Ala Leu Leu Cys Ser Asp
1               5                   10                  15

Tyr Gln Ala Asp Tyr Glu Ser
              20

```
<210> 19
<211> 15
<212> PRT
<213> artificial
```

```
<220>
<223>  Ligand of HLA-DRB1*11 (DR11)

<400>  19

Lys Ile Phe Tyr Val Tyr Met Lys Arg Lys Tyr Glu Ala Met Thr
1                   5                   10                  15


<210>  20
<211>  16
<212>  PRT
<213>  artificial

<220>
<223>  Ligand of HLA-DRB1*1501 (DR15)

<400>  20

Asp Val Gly Val Tyr Arg Ala Val Thr Pro Gln Gly Arg Pro Asp Ala
1                   5                   10                  15



<210>  21
<211>  9
<212>  PRT
<213>  artificial

<220>
<223>  MHC peptide Example 3

<400>  21

Asp Glu Tyr Ile Tyr Arg His Phe Glu
1                   5


<210>  22
<211>  10
<212>  PRT
<213>  artificial

<220>
<223>  MHC peptide Example 3

<400>  22

Ile Ala Phe Asn Ser Gly Met Glu Pro Gly
1                   5                   10
```

**Description**

FIELD OF THE INVENTION

The present invention relates to the rapid, non-diagnostic, non-therapeutic assay for MHC or MHC-correlated profiles and to the matching of such profiles as a means of determining optimal mating compatibility between individuals. Particularly, the invention is applicable for the encrypted and anonymous matching of optimal mating partners in a digital format as may be applicable in such fields as internet-based dating services. The invention includes the customization of odours (fragrances, cosmetic products) for animals, humans, objects, interior and exterior spaces based on the results of the MHC rapid assay.

BACKGROUND OF THE INVENTION

The major histocompatibility (MHC) gene complex encodes cell-surface glycoproteins (class I and II molecules) that bind peptides and present them to T lymphocytes [1]. While the MHC complex has been detected in the field of medicine, specifically in transplantation medicine, the high degree of heterozygosity in this gene complex found in natural populations of most species is most likely promoted by non-disease-based selection such as mating preferences [2]. Evolutionary biologists have been investigating MHC-dependent mating preferences in vertebrates [3] and particularly in humans [4]. Evidence is accumulating that in many species, MHC genes influence reproductive behaviour and body odour, generating MHC-correlated olfactory cues for potential mating partners [3, 5]. The high diversity of MHC genes may be responsible for the distinctive scent of individuals, which is analogous to a signature or fingerprint [6]. Such MHC-correlated odour fingerprints consist of various volatile odour compounds and MHC peptide ligands [7].
Empirically, in the three spined stickleback fish, evolutionarily conserved structural features of MHC peptide ligands were used to evaluate MHC diversity of prospective mating partners through MHC-correlated olfactory cues [8]. In human behaviour, odour cues provide information regarding the degree of relatedness and may thus affect kin preferences and mate choice [9]. For example, it has been shown that humans can influence the hormonal balance of conspecifics through chemosignals from sweat [10]. Evidence is accumulating that olfactory cues on MHC status of individuals are also possibly enhanced through the use of perfumes. Individual preferences for fragrances seem to be determined in some way by amplifying one's own body odour that reveals a person's immunogenetics [11]. Odour cues that reveal the immunogenetic status of individuals are highly resolved. Women for example have been shown to be able to differentiate a single allele difference among male odour donors with different MHC genotypes [12]. MHC-dependent mate preferences through odour cues may even influence the psychology of sexual attraction and the number of extra-pair sexual partners in relationships [13]. Evolutionary biologist assume that the optimal MHC complement for a partner should include those alleles that provide resistance against the parasites in the current environment [14]. Theoreticians have predicted two opposing forces, namely parasite resistance and inbreeding avoidance, to result in an optimal number of genes at intermediate individual MHC diversity [15, 16]. This prediction is now supported both by experimental data [17] and a population genetic survey [18] with the three spined stickleback.
MHC linked odour cues appear at the periphery via different pathways. Soluble MHC molecules for example are known that carry allele-specific odoriferous molecules from the blood via the kidneys into the urine [5] and such molecules have been identified in the urine proteome [19], where carboxylic acids are most likely to be the odour-components linked to MHC [20]. More and more data is becoming available on odour components not only in urine but also sweat and saliva and many of these components seem to be MHC determined [6]. Volatile odorant components have been experimentally shown to activate neurons in the vomeronasal organ [21]. And among those, small peptides that serve as ligands for MHC molecules function as sensory stimuli [22]. Fish, rodents and humans have been shown to be able to differentiate particular MHC profiles by smell [20] and such differences could in principle even be detected with artificial screening technology (electronic nose) [23] [24]. Data on these ligands is rapidly accumulating [25] together with their specific binding properties to MHC molecules [26]. The following paragraph reviews the currently available methods for the screening of MHC profiles.
The screening for MHC genes or MHC correlated profiles is generally directed towards medical, diagnostic purposes, the methodologies focusing on sequencing of the MHC genes or the use of genetic markers for which often blood samples have to be collected. Invasive sampling procedures and time consuming methodologies may be among the reasons why odour correlated MHC profiles have not yet been fully exploited in the life-style market. In the medical field, however, several methodologies for screening MHC profiles other than sequencing and genotyping exist. Medical research has explored the possibility for enzyme linked immunosorbent assays (ELISA) [27] to screen not only blood but also non-invasive urine, saliva and sweat samples. Typically, in such ELISA protocols, anti-monomorphic MHC class I or class II monoclonal antibodies are attached to solid substrate beads, and promote the capture of soluble MHC molecules from serum, plasma or other human body fluids [28]. The detected MHC linked proteins were typically between 23 and 45 kD in molecular weight [29] and the amount of soluble MHC molecules in saliva, for example, ranged from 9 ng/ml to 70 ng/ml [30]. However, despite the diversity of available ELISA protocols for MHC screening that have been established [31, 32], its success for medical diagnostic purposes has been limited. Either the accuracy of the assays was insufficient for medical standards compared to sequencing and/or other serological analysis, or the diagnostic power for diseases with a demand for non-invasive screening was too low (e.g. for rheumatoid arthritis) [31]. However, ELISA based protocols, in general, seem to be the most promising candidate methods for the manufacture of a rapid test in a lateral flow setting [33, 34]. Interestingly, in the context of ELISA, it has become clear that MHC molecules themselves are not completely broken down in the body but are being excreted and can themselves act as recognition cues [29]. The aim of the present invention is to manufacture a rapid assay that could be used by individuals at home. The assay results are such that they can be added to a personal profile on an Internet platform.

Internet communities such as online dating services explore personal profiles including images and other personal attributes such as body shape [35, 36] and facial characteristics [37] to determine attractiveness [38] and to bring together individuals with similar backgrounds [39]. The market volume in this field for Europe and the US alone, is between 2.5 and 5 million paying singles using Internet dating platforms and an industry generating a sales volume of approximately 1,200 million USD per year.

US patent application 2005/0112684 A1 proposes a method of matching human beings, which comprises collecting genetic sample material from individuals and subsequently determining the individuals' MHC profiles in a genetic laboratory. Similarly, US 2007/069889 A1 and US 2007/069901 disclose matching services which may include the determination of MHC profiles. These methods, however, require that the individuals give their genetic material to an institution where it is analyzed. It is likely that the users or participants will be reluctant to give their genetic material and have it analyzed at a location where it is beyond their control.

SUMMARY OF THE INVENTION

Accordingly, it is one of the objects of the present invention to provide a user-friendly test kit for the rapid determination of an individual's Major Histocompatibility Complex (MHC) profile and for the use of such profiles for customizing odours, comprising:

at least one solid substrate in contact with a proximal sample application zone, said substrate having N conjugation zones and N distal detection zones, said conjugation zones respectively containing N labelled primary binding reagents capable of binding with an analyte to form an analyte-primary binding reagent conjugate; said distal detection zone respectively having immobilized thereunto, N unlabeled secondary binding reagents capable of binding to a migrating analyte-labeled primary binding reagent conjugate to determine the presence or absence of N analytes which are indicative of defined N-MHC antigens, wherein N is an

integer greater than 3.

Another object of the invention relates to a method for the preparation of one or more customized odour compositions, comprising the following steps:

(a) providing an MHC profile of one or more individuals, wherein said MHC profile contains information on the presence or absence of N different MHC antigens in each individual, wherein N is an integer greater than 3;
(b) for each individual, selecting at least one peptide ligand of an MHC antigen found to be expressed by said individual and/or selecting at least one peptide ligand of an MHC antigen found not to be expressed by said individual;
(c) for each individual, providing at least one compound comprising said at least one peptide ligand selected in step (b), or a fragment or derivative thereof;
(d) for each individual, mixing said at least one compound with a cosmetically acceptable excipient, diluent and/or vehicle to obtain said customized odour composition.

Yet another object of this invention is a method of matching individuals, comprising the following steps:

(a) obtaining a plurality of personal profiles from a plurality of individuals and storing said personal profiles in a database on a computer.

(b) providing a plurality of test kits to said plurality of individuals, wherein each test kit comprises a unique identifier;

(c) obtaining a plurality of encrypted MHC profiles and the respective unique identifiers from said plurality of individuals;

(d) storing said encrypted MHC profiles and the respective unique identifier in a database on a computer, wherein each encrypted MHC profile and its unique identifier is allocated to one of said personal profiles;

(e) decrypting said encrypted MHC profiles using a key related to said unique identifier, and a computer, and storing the decrypted MHC profiles in a database on a computer;

(f) calculating the pair wise distances in decrypted MHC profiles between all individuals for which decrypted MHC profiles have been stored in the database;

(g) determining the quality of match between said individuals from the pair wise distances calculated in step (f).

Another object of the invention relates to a method for the rapid determination of an encrypted MHC profile of an individual in which a test kit according to the invention is contacted with a body liquid sample containing the analyte, such that the sample permeates by capillary action from a sample application zone through the solid substrate via N conjugation zones into N detection zones, and N labelled primary binding reagents migrate with the analyte from the respective conjugation zones to the respective detection zones containing immobilized N unlabelled secondary binding reagents, the presence of analytes in the sample being determined by observing the extent to which the labelled reagents become bound in the detection zones.

Another object of the invention is the customization of odours based on MHC profiles comprising the following steps:

(a) obtaining a plurality of MHC profiles from a plurality of individuals.
(b) using said plurality of MHC profiles for the customization of odours for "enhance" and "complement" as defined in the detailed description below, based on detected analytes.
(c) Providing a plurality of said customized odours to said plurality of individuals for their use to upgrade fragrances, cosmetic products, interior and exterior spaces, surfaces such as textiles or the direct application to enhance body odour, or the application in liquid, solid or gaseous material.

It is to be understood that both the foregoing general description and the following detailed description are exemplary and explanatory and are intended to provide further explanation of the invention as claimed. Other objects and features of the invention will become apparent from the following detailed description considered in conjunction with the accompanying drawings and tables. The accompanying drawings are included solely for purposes of illustration and not as a definition of the limits of the invention. Also, the drawings are not drawn to scale, and are merely conceptual in disclosing the preferred embodiments of the invention.

BRIEF DESCRIPTION OF THE DRAWINGS

Figure 1 illustrates a process flow-chart of the processes according to one embodiment of the present invention.

Figure 2a illustrates one embodiment of a lateral flow-through assay device for rapid assay to detect the encrypted MHC profile of a user.

Figure 2b illustrates how the result from such a flow-through assay device can be added to a personal profile in an online electronic form together with a unique identifier.

Figure 3 is a process chart of matching and dating services according to one embodiment of the present invention showing in chronological order, the preferred steps usable to provide a user with a list of potential dating partners that match said user's MHC profile.

Figure 4 is an object-oriented view of matching and dating services according to one embodiment of the present invention showing the objects, methods and database tables for providing a user with a list of potential dating partners that match said user's MHC profile.

Figure 5 is a chart illustrating the the probability $P_i$ with which a test field shows a positive result and the probability p, that two users have the same MHC profile for different number of test fields on the lateral flow assay N = {1..10} in accordance with one embodiment of the present invention.

## DETAILED DESCRIPTION OF THE INVENTION

<u>Test kit</u>

The test kit according to the present invention allows for a user-friendly rapid determination of an individual's MHC profile. The term "rapid" as used herein refers to the time span between the application of a test sample and the availability of the assay result, which may range from about 1 second to about 30 minutes, preferably from about 1 minute to about 20 minutes, more preferably from about 2 minutes to about 15 minutes, most preferably from about 3 to about 8 minutes. The rapid assay can in principle be any device, kit or test format that allows the detection of MHC components within the above mentioned time span. For instance, the rapid assay may be a device based on lateral flow immunochemical technique, semiconductor technique, or any other technical device able to rapidly detect MHC compounds or a correlate thereof.

The term "user-friendly" as used herein refers to the non-requirement of any specialized skills or technical training in order to use the the test kit of the invention. The test kit of the invention is suitable for use in home and is intended to give a result which is rapid and which requires the minimum degree of skill and involvement from the user. The test kit preferably requires that some portion of the kit is contacted with the sample (e.g. a urine stream or saliva) and thereafter, no further actions are required by the user before a result can be observed. Ideally, the analytical result is observable within a matter of minutes following sample application, e.g. 30 minutes or less, preferably 10 minutes or less.

The test kit of the invention is suitable for rapidly determining an individual's MHC profile. The term "MHC profile" as used herein refers to a pattern consisting of the presence or absence of a plurality of different MHC antigens expressed by an individual.

The terms "MHC antigen" (Major Histocompatibility Antigen) and "HLA antigen" (human leukocyte-associated antigen) are used interchangeably herein. Unless specified otherwise herein, the term "MHC antigen" refers to serologically defined HLA antigens. MHC antigens in the sense of this invention include, but are not limited to, HLA-A, -B, -C, -DR and -DQ antigens as defined in [40] in Tables 1 to 6 as "WHO assigned type". The term "MHC antigens" further includes HLA antigens corresponding to the alleles listed in the Scientific Database at http://www.anthonynolan.org.uk/HIG/lists/class1list.html or http://www.anthonynolan.org.uk/HIG/lists/class2list.html. Suitable MHC antigens may also be found in [45] or at the URL http://www.allelefreguencies.net.

The MHC profile determined in accordance with this invention may contain information on N different MHC antigens, wherein N is an integer greater than 3. The inventors have found that it is possible to obtain sufficient information on an individual's MHC profile by analyzing a limited number of MHC antigens. Accordingly, N is preferably an integer from 4 to 40, more preferably from 5 to 33, more preferably from 6 to 30, more preferably from 6 to 25, even more preferably from 7 to 20, most preferably from 8 to 15, or from 8 to 12 (e.g. 8, 9, 10, 11 or 12).

In particular, the inventors applied a unique frequency-based selection technique for selecting suitable MHC antigens to be included in the MHC profile. Preferably, the set of MHC antigens to be determined contains MHC antigens which are present in the population at a frequency of from about 10% to about 90%. More preferably, the frequency of the MHC antigen in the population ranges from about 15 % to about 85 %, more preferably from about 20 % to about 80 %, most preferably from about 25 % to about 75 %. In one embodiment, all MHC antigens to be determined with the test kit of the invention show this frequency. By selecting MHC antigens according to the frequency in the population, a significant result can be obtained without having to analyse a vast number of possible MHC alleles on the level of the genome. The frequency of an MHC antigen is defined as the proportion with which a given antigen can be detected in a given population. A population can, for example, be a geographically defined group of individuals, for example human individuals. Preferably, the frequency of an MHC antigen is identical to the average phenotype frequency determined on the basis of data from [45] and/or http://www.allelefrequencies.net.

Suitable antigens include, but are not limited to, those listed in Tables 1 and 2. In one embodiment, the MHC antigens to be analyzed by the test kit of the invention are selected from the group consisting of HLA-A*01, HLA-A*02, HLA-A*03, HLA-A*11, HLA-A*24, HLA-A*26, HLA-A*30, HLA-A*31, HLA-A*68, HLA-B*07, HLA-B*08, HLA-B*15, HLA-B*35, HLA-B*40, HLA-B*44, HLA-B*51, HLA-Cw*01, HLA-Cw*03, HLA-Cw*04, HLA-Cw*07, HLA-DPB1*0201, HLA-DPB1*0301, HLA-DPB1*0401, HLA-DQA1*0101, HLA-DQA1*0102, HLA-DQA1*0201, HLA-DQA1*03, HLA-DQA1*0301, HLA-DQA1*05, HLA-DQA1*0501, HLA-DQA1*0505, HLA-DRB1*01, HLA-DRB1*03, HLA-DRB1*0301, HLA-DRB1*04, HLA-DRB1*07, HLA-DRB1*0701, HLA-DRB1*11, HLA-DRB1*13, HLA-DRB1*15, and HLA-DRB1*1501. In another embodiment, the set of MHC antigens to be analyzed comprises both class I and class II MHC antigens.

In order to determine whether or not an individual has a certain MHC antigen (i.e. whether or not he/she expresses it), the presence or absence of an analyte in a test sample from said individual is detected in accordance with this invention.

The term "test sample" refers to a material suspected of containing the analyte. The test sample may, for instance, include materials derived from a biological source, such as a physiological fluid, including, saliva, sweat, urine, blood, interstitial fluid, plasma, ocular lens fluid, cerebral spinal fluid, milk, ascites fluid, mucous, synovial fluid, peritoneal fluid, vaginal fluid, menses, amniotic fluid or the like. The body fluid may be diluted or processed prior to applying it in the test kit.

As used herein, the term "analyte" refers to the substance to be detected. The analyte which is suspected of being present in the test sample and which is to be detected may be any substance indicative of a specific MHC antigen.

MHC molecules, their fragments, degradation products of their peptide ligands and products of MHC-dependent microflora have all been considered as potential odorants. The most significant contacts between peptides and the MHC molecules are mediated through the side chains of so-called anchor residues of the peptide fitting into pockets of the MHC molecules. The range of peptides displayed by the MHC molecules of an individual mirrors the structural diversity of its MHC alleles. Different peptide ligands of one particular MHC molecule share common residues (so-called anchors) whose side chains fit into the characteristic binding pockets of the MHC molecule [25]. This suggests that anchor residues could be the defining feature of peptides and olfactory assessment might focus on them. MHC-peptide complexes are shed from the cell surface and their fragments appear in serum, saliva, sweat and urine [41]. The molecular weight for MHC membrane proteins and peptide ligands found in human saliva is in the range of 40 kD [12] and similar proteins have been detected in urine and sweat. Soluble HLA molecules between 45 kD (class I heavy chain) and 23 kD have been found in urine and 45 kD and 40 kD soluble HLA associated molecules in sweat.

Preferably, the analyte is selected from the group consisting of MHC molecules, fragments of MHC molecules, MHC ligands, fragments of MHC ligands, and combinations thereof. For example, the analyte may be a peptide ligand which can be bound by its respective MHC molecule in the molecular groove. Alternatively, the peptide may already have been partially degraded resulting in fragments which may be detected as well. In body fluids such as saliva and urine, full length MHC molecules may be present. Usually, however, fragments of MHC molecules such as the extracellular portion thereof or fragments thereof are present in the test sample. It is also possible to detect a combination of MHC molecule and its ligand bound thereto. For instance, analytes may be MHC (major histocompatibility) components. These compounds may be part of the MHC gene complex or correlated to it and may include MHC-molecules, MHC-ligands, MHC-correlated peptides, MHC-correlated odour components, MHC-derived odour components, MHC-correlated carboxylic acids or antibodies to any of the above substances. In a particular embodiment, the analyte is selected from the group consisting of MHC molecules, fragments of MHC molecules, naturally occurring derivatives of MHC molecules, MHC ligands, fragments of MHC ligands, naturally occurring derivatives of MHC ligands and combinations thereof.

The test kit of the invention comprises a plurality of different pairs of binding reagents; usually it comprises N mobilizable labelled primary binding reagents and N immobilizable unlabelled secondary binding reagents, wherein N is an integer as defined hereinabove. The binding reagent may be any molecule capable of binding to the analyte. Preferably, the binding reagent is an antibody, e.g. a monoclonal antibody or a polyclonal antibody. As used herein, the term "antibody" is meant to include intact molecules as well as antibody fragments (such as, for example, Fab and F(ab')$_2$ fragments) which are capable of specifically binding to the analyte. Fab and F(ab')$_2$ fragments lack the F$_c$ fragment of intact antibody. Moreover, antibodies in accordance with the present invention include single chain antibodies. Other binding reagents such as aptamers may also be envisaged as long as they are capable of specifically binding to the analyte.

Tables 1 and 2 respectively show selection of MHC class I and class II alleles with corresponding peptide ligands to obtain the desired result for individual MHC profiles from lateral flow assay. Bold letters within selected ligands mark anchor residues likely to bind to groves of the corresponding MHC membrane protein.

MHC class I antigen-antibody conjugates have been produced for rats (A$^c$ with YR5/12 and A$^{av1}$ with JY1/116) [41] and humans (class I associated proteins with 4B5.1 monoclonal primary antibody and IgG phosphatase-conjugated goat anti-mouse or anti-rabbit secondary antibody) [29]. Suitable antibodies specifically recognizing the analyte can be generated or otherwise obtained by the skilled person. Suitable techniques for generating antibodies are disclosed in, e.g., Harlow and Lane, "Antibodies, A Laboratory Manual" CSH Press 1988, Cold Spring Harbor N.Y.

Thus, for each MHC-antigen indicating analyte, two binding reagents specifically recognizing the analyte are included. This allows a more sensitive detection of the analyte. A given pair of binding reagents consists of (i) an unlabelled secondary binding reagent that is capable of specifically binding to an analyte that is indicative of a predefined MHC antigen, and (ii) a labelled primary binding reagent that is capable of specifically binding to the same analyte. In the case of antibodies as binding reagents, the two antibodies of a pair of antibodies preferably recognize different epitopes present in the analyte molecule(s). Each pair of binding reagents contained in the kit usually detects an MHC antigen different from the MHC antigens detected by the other pairs of binding reagents in the same kit. Accordingly, a test kit containing N pairs of binding reagents will allow detection of N different MHC antigens, respectively.

The test kit may be any kit or device suitable for rapidly determining an MHC profile of an individual. With the two binding reagents, the analyte can be detected in a "sandwich" or in a competition assay. The general principle of such test formats is known to the skilled person. Particularly preferred are lateral flow assays as described in e.g. EP 291 194 B1, EP 186 799 B1, or WO 2005/121800 A2. The general principles of the test kits, test devices and assay methods disclosed in US 4,956,302; US 6,372,513; US 5,770,460 and/or US 4,943,522 can be applied in the present invention. This assay format typically involves a porous media having a mobilizable labeled antibody and an immobilized unlabeled antibody partner for the analyte of interest in the biological sample. These antibodies are often referred to as the conjugate antibody and the capture antibody. The sample is added to the porous media, to allow for formation of labeled mobilizable product which moves along the porous media to contact and react with the capture antibody to form a fixed, detectable, concentrated capture antibody/analyte/detection reagent complex. Sandwich assays may include immunological assays wherein the labeled reagent and the second binding partner are both antibodies, or are both antigens, and may also include other types of molecules. For example, enzyme immunoassays (EIA) and enzyme-linked immunosorbent assays (ELISA) are types of sandwich immunoassays, in which the binding is between an antibody and an antigen, and the labeling partner is an enzyme.

Typically, these chromatographic assay devices are comprised of a porous chromatographic medium which acts as the matrix for the binding assay. The sample of interest is added directly or indirectly to one end of the medium, and is chromatographically transported to a detection reagent with which it reacts to form a labeled conjugate product, which is then transported to a test zone containing an immobilized capture reagent such as a capture antibody, in which the presence, absence, or quantity of an analyte of interest can be determined.

The test kit of the instant comprises one or more solid substrates. A preferred substrate of the present invention is a porous medium, strip or membrane. In one embodiment, the test kit comprises one solid substrate which in turn comprises said at least 3 different pairs of binding molecules. In another embodiment, the test kit comprises 2 or more solid substrates, e.g. at least 3 solid substrates, 8 to 33 solid substrates or 8 to 12 solid substrates. In a specific embodiment, the number of solid substrates in the test kit is identical to the number of pairs of binding reagents comprised in the test kit, i.e. the test kit comprises N solid substrates. Suitable solid substrate materials include, but are not limited to, cellulose, nitrocellulose, polyethylene, polyvinyl chloride, polyvinyl acetate, copolymers of vinyl acetate and vinyl chloride, polyamide, polycarbonate, polystyrene; and "bibulous" materials such as those disclosed in US Patent No. 5,770,460 treated with blocking agents, e.g. with detergents or proteins. Suitable solid substrate materials are known to one of ordinary skill in the field.

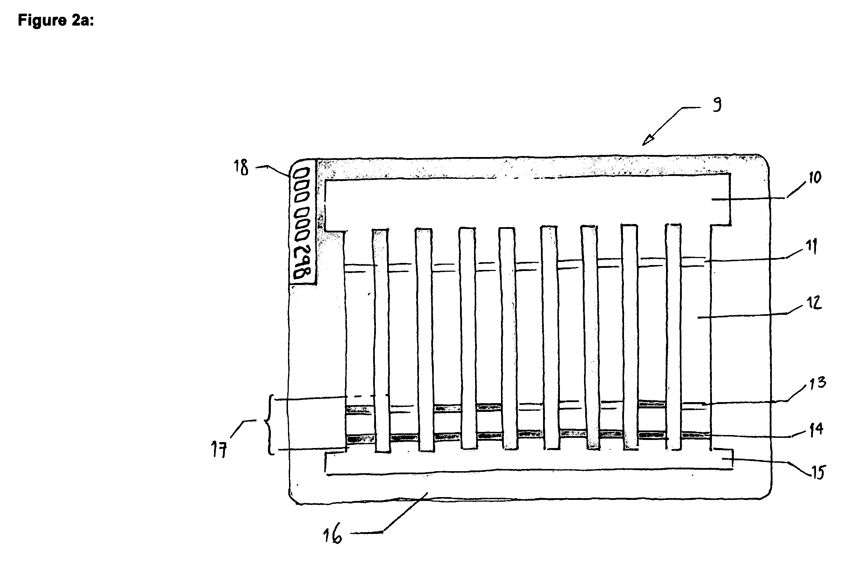

Referring to Figure 2a, the solid substrate may be a lateral flow membrane, which has on one end, a proximal sample application zone to receive a liquid sample, and further upstream, a conjugation zone wherein the analyte conjugates with a mobilizable labeled primary binding reagent, and further upstream, at least one distal detection zone having an immobilized unlabelled binding reagent affixed thereunto. The test sample contains an analyte which is an analyte which can be derivatized so as to bind the immobilized member. In one preferred configuration, the membrane is bound to two substantially fluid-impervious sheets, one on either side, with openings on one side or both sides to provide definition to the application and detection zones.

The lateral flow achieved in the kit and method of the invention is the result of the properties of the lateral flow membrane. The membrane has a much smaller thickness than surface dimension and may be hydrophilic enough to be wetted and thus permit aqueous solutions and materials to exhibit lateral flow freely, and preferably isotropically, at substantially the same rates for various components of a sample.

The membrane usually includes more than one detection zone (see Figure 2a and Example 1), usually it includes N detection zones, along with control and reference zones. Multiple detection zones are designed to detect different analytes indicative of different MHC antigens. Multiple detection zones may be in any spatial relationship to the application zone, since the membrane itself does usually not provide a barrier to sample flow.

In the method of the invention, the sample, to be analyzed for MHC-antigen indicating analytes is applied to the proximal sample application zone and allowed to be transported laterally through the membrane via a conjugation zone containing the first member of the binding reagent pair to a distal detection zone, where there is, immobilized on the membrane, the other, second member of the binding reagent pair.

In the detection zone, mobilizable binding reagent-analyte conjugate binds to the immobilized binding reagent, and the resulting bound complex is detected. Detection may use any of a variety of labels and/or markers, e.g., enzymes, liposomes, fluorescent tags, polymer dyes, or colored particles, etc., and detection is by means of, for example, direct visual observation, by developing a color, by fluorescence measurement, or by any of many other techniques by which the presence or absence of a chemical or biochemical species may be detected directly or indirectly.

The kit may be encased in a housing. The housing can be fabricated out of any convenient material (e.g. HDPE, LDPE, PP, polystyrene, acrylic, polycarbonate, etc.).

The rapid assay to which a test sample is applied is designed such that it can measure a set of different analytes that are present within one test sample. The number of different analytes may be N as defined hereinabove, preferably it is 8 - 12. Each detected analyte contributes to the assay result.

It is preferred that the test kit of the invention comprises an item or article on which a unique identifier is recorded. The unique identifier may be any information suitable to uniquely characterize the test kit. The unique identifier may be a number such as a serial number consisting of several digits (for example 5, 6, 7, 8, 9, 10, 11 or 12 digits), a barcode or a graphic representation which can be identified by a scanner.

The item on which the unique identifier is recorded may be any article, product or the like suitable to carry such information. It is preferred that the unique identifier is imprinted on said item or article. Preferably, the item or article is made of paper or cardboard. In another preferred embodiment, the unique identifier is imprinted on the solid substrate of the test kit. In yet another preferred embodiment, the unique identifier is imprinted on a housing in which the solid substrate is encased. To obtain an encrypted MHC profile, the configuration of the sensors, the signals or the substrates in the rapid assay may be encrypted. The key, which is related to said unique identifier, together with the encrypted MHC profile yields a decrypted MHC profile. The unique identifier may be a serial number or a random number without replacement or any other unambiguous number. This number may be indicated on the rapid assay.

<u>Method of matching individuals</u>

One embodiment of the present invention comprises the steps of obtaining a plurality of personal profiles from a plurality of individuals and storing said personal profiles in a database on a computer. The personal profile may encompass contact data such as postal address, telephone number, e-mail address of the individual. The personal profile may further include a photograph or image of the individual as well as personal attributes and preferences (e.g. height, colour, gender, weight, activities and the like). The personal profiles may be entered by the individual into a form on a computer screen provided by a first service provider, e.g. an internet dating service. There may be several fields which must be filled and further fields which may optionally be filled. By confirming the entry of his/her personal profile by clicking on a virtual button on the computer screen, the personal profile is transmitted to a first service provider which may be the dating service. Once the personal profile is received by the service provider, it is stored in a database on a computer. Known forms of suitable databases are known of ordinary skill in the field.

Alternatively, the personal profile may be transmitted by e-mail, by normal mail, via telefax or over the telephone.

In a further step, a test kit for rapidly determining an individual's MHC profile is provided to those individuals who have already provided their personal profiles. Alternatively, the test kit may be offered to interested individuals who have not yet provided their personal profiles. If interested, they may use the kit, determine their encrypted MHC profile and enter the results together with their personal profiles into the platform of the dating service, or enter the encrypted MHC profile in the personal data separately.

Preferably, the test kit is a test kit according to the present invention as described herein above.

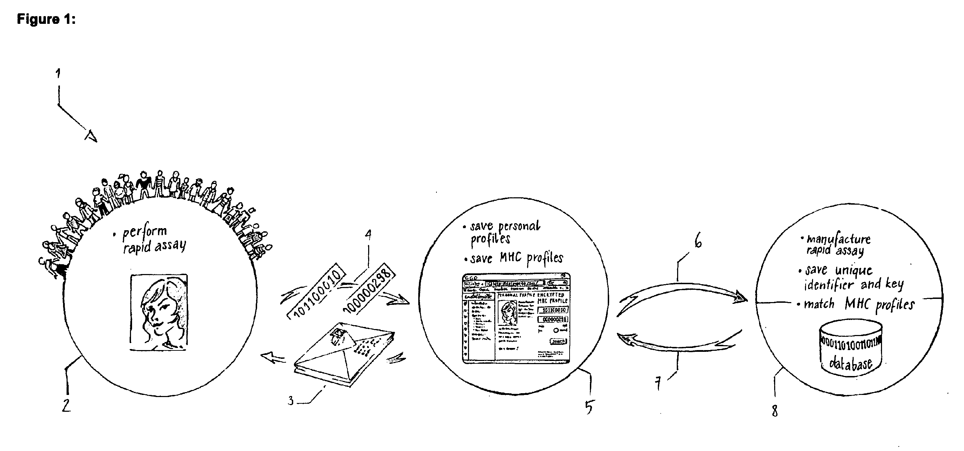

Referring to Figure 1, one embodiment of the invention can be summarized as follows: A user from a defined community 2 receives from a dating service a rapid assay 3 to detect an encrypted MHC profile. Said user performs the rapid assay and adds the results of the rapid assay (the encrypted MHC profile) on an interface of the dating service 4 to the personal profile of said user together with a unique identifier indicated on the rapid assay. The dating service 5 sends the results and the unique identifier 6 to a matching service 8. The matching service converts the assay results using a key, which is related to said unique identifier, into a decrypted MHC profile. The pair wise distance between decrypted MHC profiles is then converted into a quality of match, where intermediate distances between decrypted MHC profiles constitute better matches than large or small distances. The quality of match is sent to the dating service 7 from where said user can acquire the quality of match between said user and any other user with an account at the dating service and a valid entry for the assay result and unique identifier. It should be understood, that the company shipping the rapid assay to the user is preferably different from the matching service but can be identical to the dating service or to any other service. The company shipping the rapid assay to the user may even be the manufacturer.

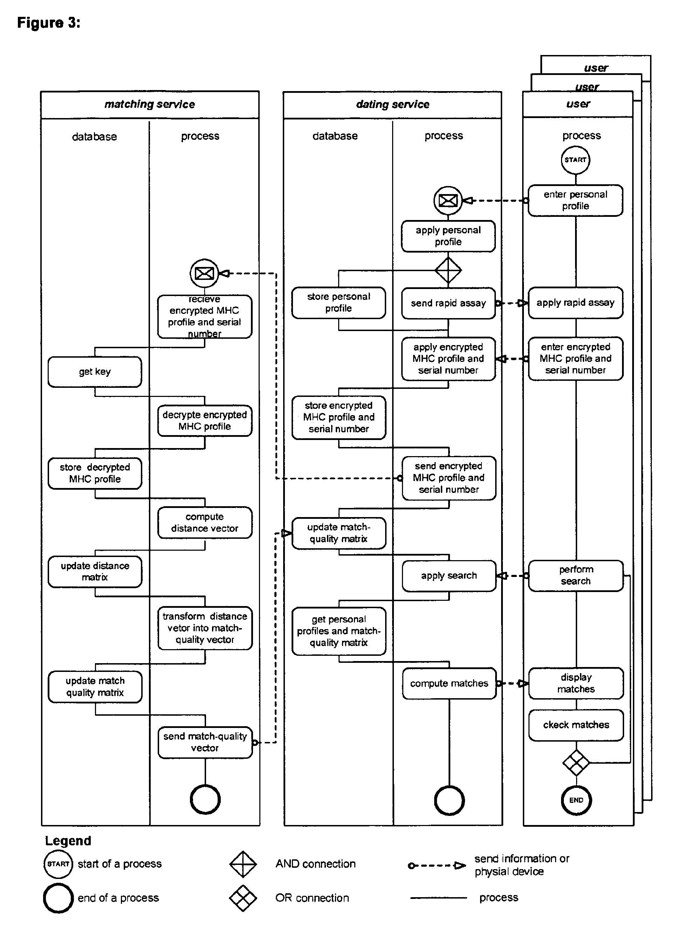

Figure 3 is a process chart of matching and dating services according to one embodiment of the present invention showing in chronological order, the preferred steps usable to provide a user with a list of potential dating partners that match said user's MHC profile.

Together with the encrypted MHC profile, the respective unique identifier is transmitted from the user to the first service provider. Accordingly, the entry form on the computer screen provided by the dating service is preferably adapted to allow the entry of two pieces of information, namely (1) the encrypted MHC profile and (2) the unique identifier. Preferably, the filling of both fields is mandatory. Entry of only one of both pieces of information would not allow transmission of the data.

The encrypted MHC profiles and the respective unique identifiers are stored in a database, preferably in a database on a computer. Each encrypted MHC profile and its corresponding unique identifier is allocated to one of the personal profiles.

The encrypted MHC profiles are decrypted using the decryption key, which is related to said unique identifier, preferably by the aid of a computer. The thus decrypted MHC profiles are than stored in a database, preferably in a database on a computer.

The matching of the decrypted MHC profiles involves two steps. Namely the step of calculating a distance measure and the transformation of said distance measure into a quality of match. The distance measure is a metric or distance function which defines a distance between elements of a set. Here we refer to the mathematical definition. Specifically a metric on a set X is a function $d : X \times X \to R$ (where R is the set of real numbers). For all x, y, z in X, this function is required to satisfy the following conditions:

1. $d(x,y) \geq 0$
2. $d(x, y) = 0$ if and only if $x = y$
3. $d(x, y) = d(y, x)$
4. $d(x, z) \leq d(x, y) + d(y, z)$

The distance measure may for instance be a Euclidian distance, a Mahalanoby distance or any other metric suitable for the MHC resolution addressed by the detected analytes. The distance measure may include the phylogeny of the MHC genes coding for the detected analytes such as the measures for example developed by or based on the following authors [42-44].

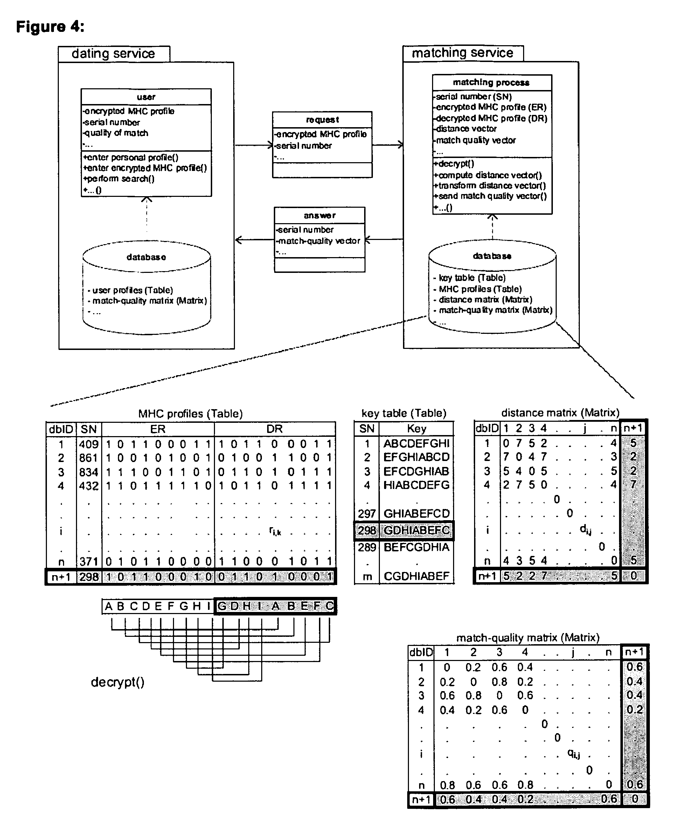

The next step thus comprises calculating the pairwise distances in decrypted MHC profiles between all individuals for which decrypted MHC profiles have been stored in the database. Theses results can be used to generate a matrix that contains the quality of match for each pairwise distance as depicted in Figure 4. From the pairwise distances, the quality of match between individuals can be calculated, preferably using a computer.

The quality of match may be defined as being "high" or "low". The quality of match between two individuals can be defined as "high" if more than 10 % and less than 90 % of the MHC antigens in the MHC profiles of the two individuals are the same. Preferably, the quality of match is regarded as "high" if more than 15 % and less than 85 % of the MHC antigens in the MHC profiles of the two individuals are the same. More preferably, the quality of match is regarded as "high" if more than 25 % and less than 75 % of the MHC antigens in the MHC profiles of the two individuals are the same.

The method of the invention may further comprise the step of providing to a first individual contact data of further individuals whose MHC profiles have a high quality of match with the MHC profile of said first individual. These data are usually provided only upon request by the first individual. The request may be submitted "online" by clicking on a virtual button or link on the computer screen.

In a specific embodiment, steps (a), (b), (c) and (d) are carried out by a first service provider while steps (e), (f) and (g) are carried out by a second service provider. That is, the decryption of the MHC profile is carried out by a different entity than that having contact with the user. For example, steps (e), (f) and (g) may be carried out by a so called matching service whereas steps (a) through (d) are carried out by the dating service which offers to provide a match between individuals.

<u>Method for preparing customized odour compositions</u>

The same MHC profiles can also be used to manufacture customized odours for a given user. The method comprises providing or determining an MHC profile of one or more individuals. The number of individuals is not particularly limited, it may be 1 or more, e.g. at least 10 or at least 100, for example 10 to 10000 or 100 to 1000. Usually, one customized odour composition is prepared for each individual for which an MHC profile has been provided or determined. The determination of the MHC profile can be carried out as described hereinabove, e.g. using the test kit described herein. Other kits and techniques can be employed as well. The MHC profile contains information on the presence (expression) or absence (no expression) of N different MHC antigens in the individual. N has the same meaning as defined above with respect to the test kit of this invention. The MHC antigens are as defined above with respect to the test kit of this invention.

For each individual, at least one peptide ligand of an MHC antigen found to be expressed in the individual is selected. That is, if the individual is positive for a certain MHC antigen, the peptide ligand of this MHC antigen is identified as described herein (preferably using the program available at <u>www.syfeithi.de</u>). After having identified it, the peptide ligand is selected according to the method of this invention.

Alternatively, or additionally, at least one peptide ligand of an MHC antigen found not to be expressed in the individual is selected. That is, if the individual is negative for a certain MHC antigen, the peptide ligand of this MHC antigen is identified as described herein (preferably using the program available at <u>www.syfpeithi.de</u>). After having identified it, the peptide ligand is selected according to the method of this invention.

For each individual, a compound is provided which comprises (1) the selected peptide ligand, (2) a fragment of the selected peptide ligand, or (3) a derivative of the selected peptide ligand. The compound preferably is a peptide. It may, however, also be a compound other than a pure peptide consisting of amino acids, e.g. it may be a peptide coupled to non-amino acid structures. The fragment of the peptide comprised in the compound preferably consists of at least 5, more preferably at least 7, still more preferably at least 10, most preferably at least 12 consecutive amino acids of the amino acid sequence of the selected peptide. The compound may comprise 1 or 2 or more (e.g. 3, 4, 5, 6, 7, 8, 9 or 10) foreign amino acids not present in the selected peptide. These foreign amino acids may be fused to the N-terminus or to the C-terminus of said fragment of the selected peptide. The derivative of the selected peptide may be an acylated, (e.g. acetylated), amidated, esterified form of the selected peptide or any other derivative including, but not limited to, oxidized forms, reduced forms, peptides coupled to a carrier or linker etc. Preferred embodiments of the derivatives are peptides, in which 1 to 10 (e.g., 1, 2, 3, 4, 5, 6, 7, 8, 9 or 10) amino acid residues of the selected peptide are substituted, added or deleted, in any combination. Particularly preferred are substitutions, additions, and deletions that do not alter the properties or activities of the selected peptide. The substitutions may be conservative amino acid substitutions, whereby a residue is substituted by another with like characteristics. Typical conservative substitutions are among Ala, Val, Leu and Ile; among Ser and Thr; among the acidic residues Asp and Glu; among Asn and Gln; and among the basic residues Lys and Arg; or aromatic residues Phe and Tyr. Preferred embodiments are peptide derivatives that retain substantially the same biological function or activity as the selected peptide.

Three properties can increase the storage life and the adaptability of such custom odour stock solutions:

1) The chosen peptides is preferably smaller than 20-mers.
2) Peptides containing Cystein or Methionin tend to oxidate and may be avoided
3) There may be constraints concerning the synthesis of the peptides e.g. distribution of amino acids with positive charge (K, R, H) etc.

Customization can be classified into "enhance" and "complement". A custom odour for "enhance" is a mixture of any number of the MHC peptides corresponding to the detected analytes on the rapid assay for said user. A custom odour for "complement" comprises MHC peptides matching the detected analytes of said user with a desired degree of matching quality as defined hereinabove or in example 3. For instance, matching could denote peptides corresponding to MHC profiles with a quality of match of 70 percent to the profile of said user. The concept of "enhance" and "complement" is explained in more detail in the example 3, with reference to the metrics and the MHC profiles used in this application.

When a "enhance" odour composition is to be provided, the method comprises selecting peptide ligands of a least 90%, preferably of at least 95%, most preferably at all of the MHC antigens found to be expressed in said individual. Similarly, not more than 10 %, preferably less than 10%, more preferably less than 5%, most preferably none of the MHC antigens found not to be expressed by said individual should be selected.

When an odour composition of the type "complement" is to be provided, the method may comprise selecting peptide ligands of 25 to 75% of all MHC antigens found to be expressed in said individual, and selecting 25 to 75% of all MHC antigens found not to be expressed in said individual.

Customized odours can contain single peptides or mixtures of the peptides mentioned above. Customized odours can be the odorant itself or they can be used as stock solutions to supplement existing fragrances. Customized odours can be added to existing fragrances, cosmetic products and even food items and beverages. For instance matching could denote peptides corresponding to MHC profiles with a quality of match of 70 percent of said user. The customized odour can contain one single peptide or any number of peptides in any combination matching said user.

Preferably, "providing the compound" includes "preparing the compound", more preferably it includes "synthesizing the compound". This can be done by solid phase peptide synthesis according to techniques known in the art. The corresponding peptides may be synthesized by standard procedures in a purity of at least 70%, preferably at least 75% more preferably at least 80%. Preferably the peptide is dissolved in water. Some peptides may require adding $NH_4OH$, urea, guanidine-HCL in deionised water depending on the overall charge of the peptide. Handling and storage of peptides is following standard procedure (described for example in the SIGMA-ALDRICH Guidelines 2007).

The invention further relates to an odour composition obtainable as described herein. This composition may comprise 1, 2, 3 or more different peptides. The length of the peptides is at least 7 amino acids, preferably 8 to 30, more preferably 10 to 25, more preferably 12 to 20, most preferably 15 to 19 amino acids.

The concentration of the peptides in the composition may range from $1 * 10^{-3}$ to $1 gL^{-1}$, preferably from 0.01 to $0.8 gL^{-1}$, most preferably from 0.1 to $0.3 gL^{-1}$. The odour compositions may be used to enhance one's own body odour ("enhance").

A preferred embodiment of the invention relates to the fields of cosmetics, particularly the manufacture of a perfume. The bodily odour can be influenced in a predetermined manner by means of adding the compositions of the invention to perfumes. Cosmetic compositions according to the invention contain common cosmetic components, such as further active substances, carriers, diluents and/or adjuvants, apart from the MHC peptide ligands. For example the cosmetic compositions may be formulated as liquids, gels, creams, slurries, ointments, aerosols, sprays, suspensions, powders etc. together with other ingredients, e.g. further odorants, auxiliaries, carriers and/or diluents. The compositions may be administered topically, orally or nasally or by any other suitable means. Preferably, the compositions are applied to skin and/or clothes of an individual in a manner that allows access of the compound to the environment, e.g. air space, enclosing the individual. The concentration of the active substance in the cosmetic compositions preferably is preferably in the subnano to micromolar range and more preferably within the range of from 1 pM to 100 $\mu$M, preferably from 10 pM to 10 $\mu$M, more preferably from 0.1 nM to 10 $\mu$M, most preferably from 1 nM to 1 $\mu$M.

The following examples further illustrate the invention. The invention, however, is not limited to these exemplary embodiments.

EXAMPLES

**Example 1: Predicting the matching quality between users based on an MHC flow-through assay**

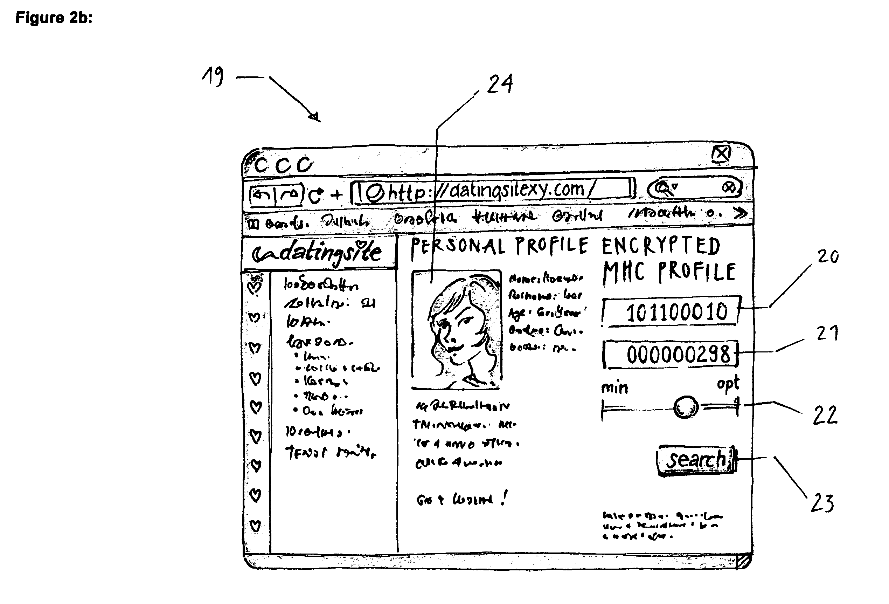

In a first step, the user enters the personal profile into an online form. The personal profile comprises among other things the postal address of said user. Referring to Figure 2b, for instance, it may also comprise a personal photograph 24. The personal profile of said user is then sent to the dating service. The dating service stores the personal profile into its database and sends a rapid assay kit to the postal address of said user.

A user performs the rapid assay. Referring to Figure 2a, for instance, one embodiment of a rapid assay in our invention may be a flow-through assay device 9. To initiate the flow through assay, a user directly applies approximately 5 ml of saliva sample to a sample pad 10 through which it will travel in the direction of the wicking pad 15. In the conjugate zone 11, a gold colloid labelled primary antibody will bind to the analyte. The superfluous conjugate will travel further through the porous membrane made of nitrocellulose 12 to the detection region 13, where a secondary antibody binds to the conjugate. The primary and the secondary antibodies are designed and manufactured such that they detect MHC antigens with two epitopes. The sensitivity of the antibodies to detect MHC antigens in the saliva sample is approximately 50 ng ml$^{-1}$. Superfluous conjugate moves further to the control region 14 where an anti-species antibody binds to the conjugate. The rest of the conjugate will be absorbed by the wicking pad 15, which promotes capillary action and fluid flow through the porous membrane 12. Staining in the detection region 13 and the control region 14 together indicate the presence or absence of each of the nine analytes for which the flow through device has been designed. A serial number 18 is indicated on the rigid material 16 containing the device 9. Presence and absence of each of the nine analytes together constitute an encrypted MHC profile of the user.

Referring to Figure 2b, the user enters the encrypted (ER) MHC profile 20 and the serial number 21 into the online form and sends it to the dating service 23. The dating service stores the encrypted MHC profile and the serial number of said user and sends it to the matching service.

The matching service receives the encrypted MHC profile and the serial number of said user. In order to decrypt the encrypted MHC profile of said user, the key to the corresponding serial number is sorted out from the key table in Figure 4 and applied to the encrypted MHC profile of said user, which yields the decrypted (DR) MHC profile of said user. Then the decrypted MHC profile of said user is stored in the MHC profiles table (see Figure 4). In the next step, the distance-vector of said user to the decrypted MHC profiles of all other users is computed and the distance matrix in Figure 4 is updated with this distance vector.

The distance vector of said user is then transformed into a match-quality vector of said user. To compute the match-quality vector of said user, a function is applied to transform the distances of the distance-vector to match-quality parameters between 0 and 1 where 0 represents the lowest and 1 the best match-quality. The matrix that contains the quality of match is then updated with the vector that contains the quality of match of said user to all other users. Then the matching service sends the match-quality vector of said user to the dating service in order to protect said user's privacy, by separating said user's personal profile from said user's decrypted MHC profile. The dating service receives the vector of said user and updates its matrix.

Referring to Figure 2b, in the next step said user enters the desired matching quality 22 and other preferences into the online form and sends the search criteria to the matching service 23. The search criteria are then processed and the necessary information is retrieved from the database of the matching service. Then the matches are computed and send to said user where they are displayed. Said user checks the matches and then either ends the search for a dating partner or performs a new search.

By way of example two possible ways how to calculate the "match-quality matrix" on the basis of the "MHC profiles" via the "distance matrix" are indicated in the following:

$$DR_i = \left\{ r_{i,1}, r_{i,1}, \ldots r_{i,N} \right\}$$

$$d_{i,j} = \sum_{k=1}^{N} \left| r_{i,k} - r_{j,k} \right|$$

Triangle-Curve (see Example 1)

$$q_{i,j} = \begin{cases} \dfrac{1}{N} 2d_{i,j} & 0 \le d_{i,j} \le \dfrac{N}{2} \\ \dfrac{1}{N}\left(2N - 2d_{i,j}\right) & \dfrac{N}{2} < d_{i,j} \le N \end{cases}$$

Quadratic-Curve

$$q_{i,j} = \frac{4}{N^2} d_{i,j}\left(N - d_{i,j}\right),\ 0 \le d_{i,j} \le N$$

$r_{i,k}$ are single (boolean) assay results which together constitute the decrypted MHC profile DR

$d_{i,j}$ is the distance function between the decrypted MHC profiles $DR_i$ and $DR_j$

$q_{i,j}$ is the function to obtain the quality of match to be entered in the match quality matrix.

**Example 2: Selection of target antigens**

Single MHC loci can have up to approximately 300 different alleles and most of these alleles occur in small frequencies in the population. Preferably, lateral flow assay of the instant invention has several test fields (9 in Example 1) of which each has a certain probability for a positive or negative result. If the frequency of the alleles is much higher or much lower than 0.5 in the population, then the expected diversity from individual MHC profiles will not be sufficient to effectively match the profile of two or several users with each other.

To circumvent this problem, MHC class I and class II alleles with phenotypic frequencies between 0.2 and 0.8 (which corresponds to 20% - 80%) on www.allelefrequencies.net are used [45]. Other allele combinations listed in http://www.anthonynolan.org.uk/HIG/lists/class1list.html or http://www.anthonynolan.org.uk/HIG/lists/class2list.html could also be a candidate. The threshold of 0.2 and 0.8 was calculated from the expected frequency necessary to distinguish individuals with a given probability

$$p = \left(p_i^{\,2} + \left(1 - p_i\right)^2\right)^n \quad \rightarrow \quad N = \frac{\ln(p)}{\ln\left(p_i^{\,2} + \left(1 - p_i\right)^2\right)}$$

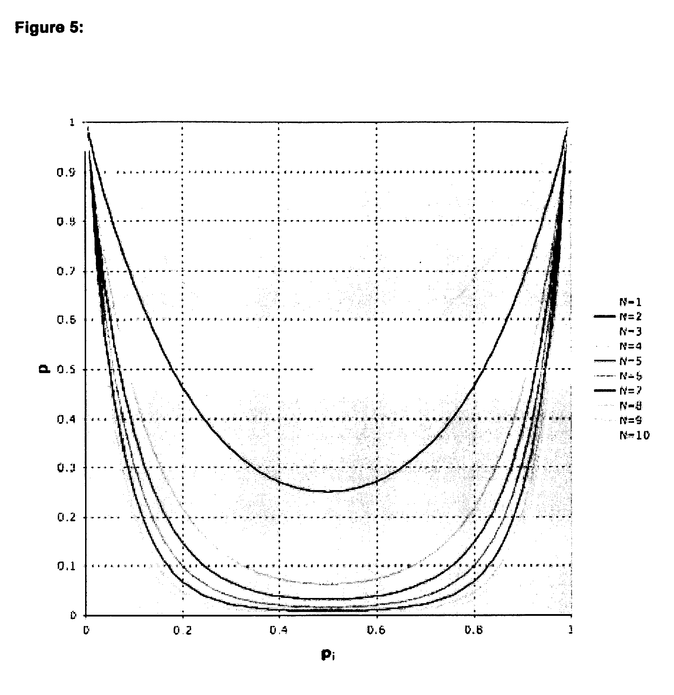

where $p_i$ is the probability with which a single test field shows a positive result, p the probability that two randomly drawn users have the same MHC profile (see Figure 5) and N the number of test fields on the assay (9 in our examples, see Figure 2a). For our examples we use $0.2 < p_i < 0.8$. The number of test fields is preferably greater than 5 with a preferred upper limit of 18 - 33.

It will be apparent to those skilled in the art that various modifications and variations can be made to the objects of the present invention without departing from the spirit or scope of the invention. Thus, it is intended that the present invention cover the modifications and variations of this invention provided they come within the scope of the appended claims and their equivalents. Accordingly, the invention is not limited by the embodiments described above which are presented as examples only but can be modified in various ways within the scope of protection defined by the appended patent claims. All documents cited herein are incorporated by reference as if included in this specification in their entireties.

**Table 1:** Selection of MHC class I alleles with corresponding peptide ligands to obtain the desired result for individual MHC profiles from the lateral flow assay. Bold letters within selected ligands mark anchor residues likely to bind to groves of the corresponding MHC membrane protein.

| MHC class | MHC allele (WHO assigned type, where available) | Average phenotype frequency in Populations with $0.2 < p_i < 0.8$ (%)[1] | Number of populations with $0.2 < p_i < 0.8$ | Selected ligand [2] | Mass of ligand (g/mol) rounded | Examples of predicted MHC epitopes | Scores for predicted epitopes[3] | Analytes used for the examples 1 and 2 (2 x 9) |
|---|---|---|---|---|---|---|---|---|
| | | | | | | | | |
| I | HLA-A*01 (A1) | 29.5 | 22 | YTDLLRLFEY | 1332 | A*01 nonamers | 22,16 | 1 |
| | HLA-A*02 (A2) | 44.2 | 65 | SLLTSSKGQLQK | 1288 | A*0201 decamers | 24,11,7 | 1 |
| | HLA-A*03 (A3) | 25.2 | 15 | | | | | |
| | HLA-A*11 (A11) | 40.0 | 17 | | | | | |
| | HLA-A*24 (A24) | 35.5 | 30 | EYPDRIMNTF | 1284 | A*0201 nonamers | 6,5 | 2 |
| | HLA-A*26 (A26) | 32.6 | 3 | ETFYVDGAANR | 1241 | A*26 nonamers | 24,5,3 | 1 |
| | HLA-A*30 (A30) | 27.6 | 7 | | | | | |
| | HLA-A*31 (A31) | 38.7 | 6 | | | | | |
| | HLA-A*68 (A68) | 23.8 | 7 | | | | | |
| | HLA-B*07 (B7) | 27.8 | 16 | VGGLKNWLVHRL | 1305 | A*0201 decamers | 25,9,2 | 2 |
| | HLA-B*08 (B8) | 25.4 | 13 | MPHEKHYTL | 1234 | B*08 octamers | 18,10 | 2 |
| | HLA-B*15 (B15) | 29.7 | 28 | | | | | |
| | HLA-B*35 (B35) | 29.9 | 24 | WASRELERF | 1192 | B*18 octamers | 13,3 | |
| | HLA-B*40 (B40) | 43.8 | 23 | REIAQDFKTD | 1221 | A*03 nonamers | 13,3 | 1 |
| | HLA-B*44 (B44) | 27.1 | 20 | DEYIYRHFF | 1288 | B*08 octamers | 14,7 | |
| | HLA-B*51 (B51) | 25.2 | 12 | DTPLIPLTIF | 1128 | A*0201 nonamers | | 2 |

(continued)

| MHC class | MHC allele (WHO assigned type, where available) | Average phenotype frequency in Populations with $0.2 < p_i < 0.8$ (%)[1] | Number of populations with $0.2 < p_i < 0.8$ | Selected ligand [2] | Mass of ligand (g/mol) rounded | Examples of predicted MHC epitopes | Scores for predicted epitopes[3] | Analytes used for the examples 1 and 2 (2 x 9) |
|---|---|---|---|---|---|---|---|---|
| | HLA-Cw*01 (Cw1) | 31.1 | 13 | | | | | |
| | HLA-Cw*03 (Cw3) | 39.6 | 26 | SAYGEPRKL | 1019 | B*08 octamer | 12,6 | 2 |
| | HLA-Cw*04 (Cw4) | 31.8 | 34 | | | | | |
| | HLA-Cw*07 (Cw7) | 45.1 | 39 | | | | | |
| | | | | | | | | |

1) Data from www.allelefrequencies.net
2) Data from www.syfpeithi.de
3) Scores are based on the frequency of the respective amino acids in natural ligands, T-cell epitopes or binding peptides. For details see Rammensee etal. (1999)

Table 2: Selection of MHC class II alleles with corresponding peptide ligands to obtain the desired result for individual MHC profiles from the lateral flow assay. Bold letters within selected ligands mark anchor residues likely to bind to groves of the corresponding MHC membrane protein.

| MHC class | MHC allele (WHO assigned type, where available) | Average phenotype frequency in Populations with $0.2 < p_i < 0.8$ (%)[1] | Number of population s with $0.2 < p_i < 0.8$ | Selected ligand [2] | Mass of ligand (g/mol) rounded | Examples of predicted MHC epitopes | Scores for predicted epitopes[3] | Analytes used for the examples 1 and 2 (2 x 9) |
|---|---|---|---|---|---|---|---|---|
| | | | | | | | | |
| II | HLA-DPB1*0201 | 33.2 | 18 | EIVDLMCHAT | 1130 | A*0201 nonamers | 12,6 | 1 |
| | HLA-DPB1*0301 | 30.1 | 10 | | | | | |
| | HLA-DPB1*0401 | 56 | 19 | IAFNSGMEPGVVAEKV | 1646 | A*03 decamers | 16,11,8,7,3,3 | 2 |
| | HLA-DQA1*0101 | 28.3 | 10 | | | | | |
| | HLA-DQA1*0102 | 34.7 | 23 | | | | | |
| | HLA-DQA1*0201 | 26.8 | 13 | QPQQPQQSFPEQERP | 1822 | B*2705 nonamers | 16,12.9,5,4,3,1 | 1 |
| | HLA-DQA1*03 | 40.5 | 14 | | | | | |
| | HLA-DQA1*0301 | 47.8 | 6 | HKLQDASAEVERLRR | 1806 | A*0201 decamers | 16,16.12,7.6.3 | 1 |
| | HLA-DQA1*05 | 48.5 | 9 | | | | | |
| | HLA-DQA1*0501 | 47.1 | 17 | | | | | |
| | HLA-DQA1*0505 | 41.1 | 5 | | | | | |

| MHC class | MHC allele (WHO assigned type, where available) | Average phenotype frequency in Populations with $0.2 < p_i < 0.8$ (%)[1] | Number of population s with $0.2 < p_i < 0.8$ | Selected ligand [2] | Mass of ligand (g/mol) rounded | Examples of predicted MHC epitopes | Scores for predicted epitopes[3] | Analytes used for the examples 1 and 2 (2 x 9) |
|---|---|---|---|---|---|---|---|---|
| | HLA-DRB1*01 (DR1) | 23.3 | 19 | | | | | |
| | HLA-DRB1*03 (DR3) | 25.8 | 20 | IPENLFLKSDGRVKYT | 1879 | A*03 nonamers | 28.16,14,7,5,3,3 | 2 |
| | HLA-DRB1*0301 (DR17) | 24.0 | 17 | ISNOLTLDSNTKYFHKLN | 2162 | DRB1*0301 15-mers | 36,17,10,3 | 2 |
| | HLA-DRB1*04 (DR4) | 30.0 | 35 | | | | | |
| | HLA-DRB1*0701 (DR7) | 28.3 | 18 | KVDLTFSKOHALLCSDYQADUES | 2660 | DRB1*0701 15-mers | 22,14,10,10.8,86,6,0 | 1 |
| | HLA-DRB1*11 (DR11) | 30.7 | 32 | KIFYVYMKRKYEAMT | 1970 | B*1501 decamers | 12,9,4,1,0,1 | 1 |
| | HLA-DRB1*13 (DR13) | 26.8 | 27 | | | | | |
| | HLA-DRB1*1501 (DR15) | 26.6 | 11 | DVGVYRAVTPQGRPDA | 1699 | DRB1*1501 15-mers | 18.8 | 2 |

(continued)

| MHC class | MHC allele (WHO assigned type, where available) | Average phenotype frequency in Populations with $0.2 < p_i < 0.8$ (%)[1] | Number of population s with $0.2 < p_i < 0.8$ | Selected ligand [2] | Mass of ligand (g/mol) rounded | Examples of predicted MHC epitopes | Scores for predicted epitopes[3] | Analytes used for the examples 1 and 2 (2 x 9) |
|---|---|---|---|---|---|---|---|---|
| | | | | | | | | |

4) Data from www.allelefrequencies.net
5) Data from www.syfpeithi.de
6) Scores are based on the frequency of the respective amino acids in natural ligands, T-cell epitopes or binding peptides. For details see Rammensee etal. (1999)

**Example 3: Customization of odours**

For this example, the decrypted MHC profile, obtained with the rapid assay as described above may correspond to positive results for the three following detected MHC peptides of said user with the sequences: EYPDRIMNTF, DEYIYRHFE and IAFNSGMEPG. Alternatively the latter three peptides could be the peptides best matching the MHC profile of said user. For the discussion below it is always assumed that detected MHC peptides of a given user reflect MHC class I or II alleles as has been described in example 2 and the tables 1 and 2 therein. Therefore, MHC profile and peptides detected in a sample of a given user are interchangeable and customization of odours could in principle be based on a genetic MHC profile of a given user (through sequencing, genetic marker analysis such as short tandem repeads etc.).

It has to be understood that although this example is limited to three peptides it can be any number of peptides from tables 1 and 2 (depending on the number of peptides for which a detection zone has been provided with the rapid assay, in the example depicted in figure 2 this would be 9 peptides). In principle, however, the list of possible peptides is not limited to those mentioned in tables 1 and 2, it can contain any known MHC peptide detected as analyte in a sample of a given user or any MHC peptide matching to detected analytes in a sample of a given user.

Now imagine said user wants to have a fragrance customized to match its own MHC profile for "enhance" or "complement". The following gives an example of how this works:

The peptides mentioned above, which can either be the peptides detected in a sample of said user or the peptides matching to the peptides detected in a sample of said user, have been synthesised with standard procedures (e.g. NMI Peptides, Reutlingen Germany). For this example, the Characteristics of synthetic peptides for the customization of odours, amino acid sequence (AS seq.), molecular weight (MW), mass, HPLC-purity, odour note as rated by participants and overall charge are given in the table below:

| Nr. | AS seq. | MW | Mass | HPLC purity | Odour Note (N = 20) | Consistency (N = 20) | Charge |
|-----|---------|------|---------|-------------|----------------------|-----------------------|--------|
| 1 | H-EYPDR IMNTF - OH | 1285,44 | 1284,58 | 77.8 | Acidic, Rubber | 0.8 | -1 |
| 2 | H-DEYIY RHFE - OH | 1289,41 | 1288,59 | 78,3 | Mild, Acidic | 0.6 | 0 |
| 3 | H-IAFNS GMEPG VVAEK V -OH | 1647 | 1646,83 | 88.2 | Mossy | 1 | -2 |

The amount of 2.125 mg of each of the three peptides was dissolved in 6 ml of deionised water. Twenty participants have classified the resulting test solution for odour note. In a blind test, odours were presented in a standardized way on 150 mm $\varnothing$ filter paper (Whatman). The average consistency with which the individual solutions could be recognized has been recorded. These solutions can be used as additive for any existing fragrance product, they can be used to produce a mixture of the synthetic peptides, which can again be applied directly to surfaces of any kind or be added to existing fragrances. Dissolved peptides provide stock solutions for any application for which the surrounding odour environment is intended to be customized to reflect "enhance" and "complement" of a given user or even a group of users.

To explain in more detail what is meant with "enhance" and "complement" we refer to the distance metrics defined in the detailed description of the invention and in example 1. Therein, the MHC profile $R_i$ of a given user $i$ comprises of a set of Boolean $\{r_{i,1}, r_{i,2}, \ldots r_{i,k} \ldots ri,N\}$ which contains the information on the detected analytes, e.g. MHC peptides $\{P_1, P_2, \ldots P_k, \ldots P_N\}$, by the rapid assay. (e.g. N = the number of analytes tested = 9 detection zones, figure 2).

The general instruction to create a customized odour for "enhance", where $\Psi_i$ is the set of indices $k$ of the result $r_{i,k}$ with a positive result, i.e. $r_{i,k}=1$, of user $i$ and $P_k$ is the MHC peptide corresponding to the detected analyte $k$ is:

$$\Psi_i = \{k \mid \forall r_{i,k} = 1\}$$

$$enhance_i = \{P_k \mid \forall k \in \Psi_i\}$$

and the general instruction to customize an odour for "complement", where $\Omega_i$ is the set of MHC profiles $R_j$ complementary to the MHC $R_i$ profile of user i with a given range for the quality of match $\Delta d$ (e.g. $\Delta d = 0$, optimal match, $\Delta d > 0$, optimal match and matches within given range), is:

$$\Omega_i = \left\{ R_j \mid (\forall j \neq i) \wedge \left( \frac{N}{2} + \Delta d < d_{i,j} \leq \frac{N}{2} + \Delta d \right) \right\}$$

$I_i$ is the set of indices $j$ of the complementary MHC profiles $R_j$ out of the set $\Omega_i$.

$$I_i = \left\{ j \mid \forall R_j \in \Omega_i \right\}$$

and $\Psi_j$ is the set of indices k of the result $r_{j,k}$ of the complementary MHC profiles $R_j$ with a positive result, i.e. $r_{j,k}=1$.

$$\Psi_j = \left\{ k \mid \forall r_{j,k} = 1 \right\}$$

the customized odour for the complementary MHC profile $R_j$ is contains the set of analytes $P_k$ with the index k of the set $\Psi_j$.

$$complement_{i,j} = \left\{ P_k \mid \forall k \in \Psi_j \right\}$$

The set of customized odours (other$_i$) complementary to the MHC profile $R_i$ is contains all customized odours (other$_{i,j}$) where $j$ is element of the set $I_i$.

$$complement_i = \left\{ other_{i,j} \mid \forall j \in I_i \right\}$$

**References**

1. Wedekind, C. and D. Penn, MHC genes, body odours, and odour preferences. Nephrology Dialysis Transplantation, 2000. 15: p. 1269 - 1271.

2. Eggert, F., et al., The major histocompatibility complex and the chemosensory signalling of individuality in humans. Genetica, 1999. 104: p. 265 - 273.

3. Penn, D.J. and W.K. Potts, The evolution of mating preferences and major histocompatibility complex. The American Naturalist, 1999. 153(2): p. 145 - 164.

4. Wedekind, C., et al., MHC-dependent mate preferences in humans. Proc. R. Soc. Lond. B, 1995. 260: p. 245 - 249.

5. Singh, P.B., Chemosensation and genetic individuality. Reproduction, 2001. 121: p. 529 - 539.

6. Penn, D.J., et al., Individual and gender fingerprints in human body odour. J. R. Soc. Interface, 2006. 4: p. 331 - 340.

7. Restrepo, D., et al., Odortypes and MHC peptides: complementary chemosignals of MHC haplotype. Trends in Neurosciences, 2006. 29(11).

8. Milinski, M., et al., Mate choice decisions of stickleback females predictably modified by MHC peptide ligands. Prodeedings of the National Academy of Science, 2005. 102(12): p. 4414 - 4418.

9. Ables, E.M., L.M. Kay, and J.M. Mateo, Rats assess degree of relatedness from human odors. Physiology & Behavior, 2007. 90: p. 726 - 732.

10. Wyart, C., et al., Smelling a single component of male sweat alters levels of cortisol in women. Journal of Neuroscience, 2007. 27(6): p. 1261 - 1265.

11. Milinski, M. and C. Wedekind, Evidence for MHC-correlated perfume preferences in humans. Behavioral Ecology, 2001. 12(2): p. 140 - 149.

12. Jacob, S., et al., Paternally inherited HLA alleles are associated with women's choice of male odor. Nature Genetics, 2002. 30(2): p. 175 - 179.

13. Garver-Apgar, C.E., et al., Major histocompatibility complex alleles, sexual responsivity, and unfaithfulness in romantic couples. PsychSc, 2006. 17(10): p. 830 - 836.

14. Milinski, M., The major histocompatibility complex, sexual selection, and mate choice. Annual Review in Ecology, Evolution, and Systematics, 2006. 37: p. 159 - 186.

15. Nowak, M.A., K. Tarczy-Hornoch, and J.M. Austy, Proc. Natl. Acad. Sci., 1992. 89: p. 10896.

16. DeBoer, R.J. and A.S. Perelson, Proceedings of the Royal Society London B, 1993.252: p. 171.

17. Reusch, T.B.H., et al., Female sticklebacks count alleles in a strategy of sexual selection explaining MHC polymorphism. Nature, 2001. 414: p. 300 - 302.

18. Wegner, K.M., et al., Parasite selection for immunogenetic optimality. Science, 2003. 301: p. 1343.

19. Zerefos, P.G., et al., Characterization of the human urine proteome by preparative electrophoresis in combination with 2-DE. Proteomics, 2006. 6: p. 4346 - 4355.

20. Singer, A.G., G.K. Beauchamp, and K. Yamazaki, Volatile signals of the major histocompatibility complex in male mouse urine. Proc. Natl. Acad. Sci., 1997. 94: p. 2210 - 2214.

21. Krieger, J., et al., Selective activation of G protein subtypes in the vomeronasal organ upon stimulation wiht urine-derived compounds. The Journal of Biological Chemistry, 1999. 274(8): p. 4655 - 4662.

22. Leinders-Zufall, T., et al., MHC class / peptides as chemosensory signals in the vomeronasal organ. Science, 2004. 306(5): p. 1033 - 1037.

23. Montag, S., et al., "Electronic nose" detects major histocompatibility complex-dependent prerenal and postrenal odor components. Proc. Natl. Acad. Sci., 2001. 98: p. 9249 - 9254.

24. Boehm, T. and F. Zufall, MHC peptides and the sensory evaluation of genotype. Trends in Neurosciences, 2006. 29(2).

25. Rammensee, H.-G., et al., SYFPEITHI: database for MHC ligands and peptide motifs. Immunogenetics, 1999. 50: p. 213 - 219.

26. Yewdell, J.W. and J.R. Bennink, Immunodominance in major histocompatibility complex class I-restircet T lymphocyte responses. Annual Review in Immunology, 1999. 17: p. 1 - 30.

27. Zavazava, N., G. Leimenstoll, and W. Muller-Ruchholtz, Measurement of soluble MHC class / molecules in renal graft patients: a noninvasive allograft monitor. Journal of Clinical Laboratory Analysis, 1990. 4: p. 426 - 429.

28. Adamashvili, I., et al., Soluble HLA: patterns of expression in normal subjects, autoimmune diseases, and transplant recipients. Rheumatol Int, 2005. 25: p. 491 - 500.

29. Wobst, B., et al., Molecular forms of soluble HLA in body fluids: potential determinants of body odor cues. Genetica, 1999. 104: p. 275 - 283.

30. Adamashvili, I., et al., Soluble HLA in saliva of patients with autoimmune rheumatic diseases. Rheumatol Int, 2002. 22: p. 71 - 76.

31. Hayman, M.W., et al., Soluble human leukodyte antigen: A diagnostic indicator of rheumatoid arthritis. Journal of Immunological Methods, 2006. 315: p. 19 - 26.

32. Amirghofran, Z., et al., Journal of Cancer Research Clinical Oncology. 128, 2002(443 - 448).

33. Utsumi, M., et al., Expression of major urinary protein genes in the nasal glands associated with general olfaction. Neurobiology, 1999. 39: p. 227 - 236.

34. Brennan, P.A. and F. Zufall, Pheromonal communication in vertebrates. Nature, 2006. 444: p. 308 -.

35.Fan, J., et al., Visual perception of male body attractiveness. Proceedings of the Royal Society London B, 2005. 272: p. 219 - 226

36. Tovée, M.J., et al., Effects of sexual dimorphism on facial attractiveness. Lancet, 1999. 399: p. 215 - 216.

37. Perrett, D.I., et al., Effects of sexual dimorphism on facial attractiveness. Nature, 1998. 394: p. 884 - 887.

38. Buss, D.M., The evolution of desire: strategies of human mating. 1994, New York: Basic Books.

39. Gourab Goshal, P.H., Attractiveness and activity in internet comunities. Physica, 2006. 364: p. 603 - 609.

40. Schreuder, G.M.T., et al., The HLA Dictionary 2004: a summary of HLA-A, -B, -C, -DRB 1/3/4/5 and -DQB1 alleles and their association with serologically defined HLA-A, -B, -C, -DR and -DQ antigens. Tissue Antigens, 2005. 65: p. 1 - 55.

41. Singh, P.B., R.E. Brown, and B. Roser, MHC antigens in urine as olfactory recognition cues. Nature, 1987. 327: p. 161 - 164.

42. Reynolds, J.B., B.S. Weir, and C.C. Cockerham, Estimation of the coancestry coefficient: basis for a short-term genetic distance. Genetics, 1983. 105: p. 767 - 779.

43. Cavalli-Sforza, L.L. and A.W.F. Edwards, Phyligenetic analysis: models and estimation procedures. Evolution, 1967. 32: p. 550 - 570.

44. Nei, M., Genetic distance between populations. American Naturalist, 1972. 106: p. 283 - 292.

45. Midleton, D., et al., www. allelefrequencies. net New allele frequency database. Tissue Antigens, 2003. 61: p. 403 - 407.

## Claims

1. A method for the preparation of one or more customized odour compositions, comprising the following steps:

(a) providing an MHC profile of one or more individuals, wherein said MHC profile contains information on the presence or absence of N different MHC antigens in each individual, wherein N is an integer greater than 3;
(b) for each individual, selecting at least one peptide ligand of an MHC antigen found to be expressed by said individual and/or selecting at least one peptide ligand of an MHC antigen found not to be expressed by said individual;
(c) for each individual, providing at least one compound comprising said at least one peptide ligand selected in step (b), or a fragment or derivative thereof;

(d) for each individual, mixing said at least one compound with a cosmetically acceptable excipient, diluent and/or vehicle to obtain said customized odour composition.

2. The method according to claim 1, wherein N is an integer from 8 to 15.

3. The method according to claim 1 or 2, wherein the frequency of said MHC antigens in the population is from 15% to 85%.

4. The method according to any one of the preceding claims, wherein step (b) comprises selecting peptide ligands of all MHC antigens found to be expressed in said individual, and not selecting any peptide ligand of MHC antigens found not to be expressed in said individual.

5. The method according to any one of claims 1 to 3, wherein step (b) comprises selecting peptide ligands of all MHC antigens found not to be expressed in said individual, and not selecting any peptide ligand of MHC antigens found to be expressed in said individual.

6. The method according to any one of claims 1 to 3, wherein step (b) comprises selecting peptide ligands of 25 to 75% of all MHC antigens found to be expressed in said individual.

7. The method according to claim 6, wherein step (b) further comprises selecting 25 to 75% of all MHC antigens found not to be expressed in said individual.

8. The method according to any one of the preceding claims, wherein step (a) comprises determining a plurality of MHC profiles of a plurality of individuals.

9. The method according to any one of the preceding claims, wherein step (c) comprises synthesizing said compound.

10. The method according to any one of the preceding claims, wherein the MHC profile is determined using a test kit for the rapid determination of an individual's MHC profile, said test kit comprising:

at least one solid substrate in contact with a proximal sample application zone, said substrate having N conjugation zones and N distal detection zones, said conjugation zones respectively containing N labelled primary binding reagents capable of binding with an analyte to form an analyte-primary binding reagent conjugate; said distal detection zone respectively having immobilized thereunto, N unlabeled secondary binding reagents capable of binding to a migrating analyte-labeled primary binding reagent conjugate to determine the presence or absence of N analytes which are indicative of defined N-MHC antigens, wherein N is an integer greater than 3.

11. An odour composition obtainable by the method according to any one of the preceding claims, comprising at least one peptide.

12. The customized odour composition according to claim 11, comprising at least two different peptides.

13. The customized odour composition according to claim 12, comprising at least three different peptides

14. The customized odour composition according to any one of claims 11 to 13, wherein the concentration of said at least one peptide in the composition is from 0.01 to 0.8 gL$^{-1}$.

15. The customized odour composition according to any one of claims 11 to 14, wherein the length of said at least one peptide is from 7 to 18 amino acids.

16. The use of the customized odour composition according to any one of claims 11 to 15 for enhancing an individual's own body odour.

17. The use of an MHC profile for the preparation of a customized odour composition.

18. The use of the customized odour composition according to any one of claims 11 to 15 for the manufacture of a perfume.

19. A method for the cosmetic treatment of an individual, comprising administering or applying to the individual the customized odour composition according to any one of claims 11 to 15.

EP 2 072 529 A1

**Figure 1:**

Figure 2a:

Figure 2b:

EP 2 072 529 A1

**Figure 3:**

**Legend**

| | | | |
|---|---|---|---|
| (START) start of a process | ◆ AND connection | ◦----▷ | send information or physial device |
| ◯ end of a process | ◈ OR connection | ——— | process |

**Figure 4:**

dating service

**user**
-encrypted MHC profile
-serial number
-quality of match
-...
+enter personal profile()
+enter encrypted MHC profile()
+perform search()
+...()

**request**
-encrypted MHC profile
-serial number
-...

**answer**
-serial number
-match-quality vector
-...

**database**
- user profiles (Table)
- match-quality matrix (Matrix)
- ...

matching service

**matching process**
-serial number (SN)
-encrypted MHC profile (ER)
-decrypted MHC profile (DR)
-distance vector
-match quality vector
-...
+decrypt()
+compute distance vector()
+transform distance vector()
+send match quality vector()
+...()

**database**
- key table (Table)
- MHC profiles (Table)
- distance matrix (Matrix)
- match-quality matrix (Matrix)
- ...

MHC profiles (Table)

| dbID | SN | ER | DR |
|---|---|---|---|
| 1 | 409 | 1 0 1 1 0 0 0 1 1 | 1 0 1 1 0 0 0 1 1 |
| 2 | 861 | 1 0 0 1 0 1 0 0 1 | 0 1 0 0 1 1 0 0 1 |
| 3 | 834 | 1 1 1 0 0 1 1 0 1 | 0 1 1 0 1 0 1 1 1 |
| 4 | 432 | 1 1 0 1 1 1 1 1 0 | 1 0 1 1 0 1 1 1 1 |
| . | . | . . . . . . . . . | . . . . . . . . . |
| . | . | . . . . . . . . . | . . . . . . . . . |
| i | . | . . . . . . . . . | . . . $r_{i,k}$ . . . . |
| . | . | . . . . . . . . . | . . . . . . . . . |
| n | 371 | 0 1 0 1 1 0 0 0 0 | 1 1 0 0 0 1 0 1 1 |
| n+1 | 298 | 1 0 1 1 0 0 0 1 0 | 0 1 1 0 1 0 0 0 1 |

ABCDEFGHI  GDHIABEFC

decrypt()

key table (Table)

| SN | Key |
|---|---|
| 1 | ABCDEFGHI |
| 2 | EFGHIABCD |
| 3 | EFCDGHIAB |
| 4 | HIABCDEFG |
| | |
| 297 | GHIABEFCD |
| 298 | GDHIABEFC |
| 289 | BEFCGDHIA |
| . | . |
| m | CGDHIABEF |

distance matrix (Matrix)

| dbID | 1 | 2 | 3 | 4 | . | . | j | . | n | n+1 |
|---|---|---|---|---|---|---|---|---|---|---|
| 1 | 0 | 7 | 5 | 2 | . | . | . | . | 4 | 5 |
| 2 | 7 | 0 | 4 | 7 | . | . | . | . | 3 | 2 |
| 3 | 5 | 4 | 0 | 5 | . | . | . | . | 5 | 2 |
| 4 | 2 | 7 | 5 | 0 | . | . | . | . | 4 | 7 |
| . | . | . | . | . | 0 | . | . | . | . | |
| . | . | . | . | . | . | 0 | . | . | . | |
| i | . | . | . | . | . | . | $d_{i,j}$ | . | . | |
| . | . | . | . | . | . | . | . | 0 | . | |
| n | 4 | 3 | 5 | 4 | . | . | . | . | 0 | 5 |
| n+1 | 5 | 2 | 2 | 7 | | | | | 5 | 0 |

match-quality matrix (Matrix)

| dbID | 1 | 2 | 3 | 4 | . | . | j | . | n | n+1 |
|---|---|---|---|---|---|---|---|---|---|---|
| 1 | 0 | 0.2 | 0.6 | 0.4 | . | . | . | . | . | 0.6 |
| 2 | 0.2 | 0 | 0.8 | 0.2 | . | . | . | . | . | 0.4 |
| 3 | 0.6 | 0.8 | 0 | 0.6 | . | . | . | . | . | 0.4 |
| 4 | 0.4 | 0.2 | 0.6 | 0 | . | . | . | . | . | 0.2 |
| . | . | . | . | . | . | 0 | . | . | . | |
| . | . | . | . | . | . | . | 0 | . | . | |
| i | . | . | . | . | . | . | $q_{i,j}$ | . | . | |
| . | . | . | . | . | . | . | . | 0 | . | |
| n | 0.8 | 0.6 | 0.6 | 0.8 | . | . | . | . | 0 | 0.6 |
| n+1 | 0.6 | 0.4 | 0.4 | 0.2 | | | | | 0.6 | 0 |

**Figure 5:**

**European Patent**
**Office**

**PARTIAL EUROPEAN SEARCH REPORT**

**Application Number**

which under Rule 63 of the European Patent Convention EP 07 02 5035
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2007/243537 A1 (TUCK EDWARD F [US] ET AL) 18 October 2007 (2007-10-18)<br><br>* abstract *<br>* paragraph [0099] *<br>* paragraphs [0113] - [0115] *<br>* figure 21 *<br>----- | 1,2,<br>16-19<br>3-15 | INV.<br>C07K14/74<br>G01N33/52<br>C11B9/00 |
| Y | US 4 943 522 A (EISINGER ROBERT W [US] ET AL) 24 July 1990 (1990-07-24)<br>* abstract *<br>* claim 1 *<br>----- | 10 | |
| Y | EP 1 358 869 A (MAX PLANCK GESELLSCHAFT [DE]) 5 November 2003 (2003-11-05)<br>* example 1.2 *<br>* paragraphs [0006] - [0008] *<br>* claims 1-14 *<br>----- | 3-15 | |
| A | US 2005/112684 A1 (HOLZLE ERIC [US]) 26 May 2005 (2005-05-26)<br>* figure 1 *<br>* claims 1-3 *<br>-----<br>-/-- | 1-19 | |

**TECHNICAL FIELDS SEARCHED (IPC)**

C07K
G01N
C11B

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 9 May 2008 | Jones, Laura |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.....................................................................................
& : member of the same patent family, corresponding
document

EPO FORM 1503 03.82 (P04E07)

EP 2 072 529 A1

**European Patent Office**     **PARTIAL EUROPEAN SEARCH REPORT**     Application Number

EP 07 02 5035

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| A | MILINSKI M ET AL: "EVIDENCE FOR MHC-CORRELATED PERFUME PREFERENCES IN HUMANS" BEHAVIORAL ECOLOGY, XX, XX, vol. 12, no. 2, 1 January 2001 (2001-01-01), pages 140-149, XP001105765 ISSN: 1045-4455 * the whole document * ----- | 1-19 | |
| A | US 2003/087796 A1 (ZIEGLER ANDREAS [DE] ET AL) 8 May 2003 (2003-05-08) * abstract * * paragraph [0005] * * claim 1 * ----- | 1-19 | |

TECHNICAL FIELDS SEARCHED (IPC)

EPO FORM 1503 03.82 (P04C10)

36

Although claim 19 is directed to a method of treatment of the human/animal body (Article 53(c) EPC), the search has been carried out and based on the alleged effects of the compound/composition.

-----

Claim(s) not searched:

-

Reason for the limitation of the search (non-patentable invention(s)):

Article 53 (c) EPC - Method for treatment of the human or animal body by therapy

**ANNEX TO THE EUROPEAN SEARCH REPORT
ON EUROPEAN PATENT APPLICATION NO.**                    EP 07 02 5035

This annex lists the patent family members relating to the patent documents cited in the above-mentioned European search report.
The members are as contained in the European Patent Office EDP file on
The European Patent Office is in no way liable for these particulars which are merely given for the purpose of information.

09-05-2008

| Patent document cited in search report | | Publication date | Patent family member(s) | | Publication date |
|---|---|---|---|---|---|
| US 2007243537 | A1 | 18-10-2007 | NONE | | |
| US 4943522 | A | 24-07-1990 | NONE | | |
| EP 1358869 | A | 05-11-2003 | AU | 2003227677 A1 | 10-11-2003 |
| | | | CA | 2483750 A1 | 06-11-2003 |
| | | | WO | 03090705 A1 | 06-11-2003 |
| | | | JP | 2005532299 T | 27-10-2005 |
| | | | US | 2006039883 A1 | 23-02-2006 |
| US 2005112684 | A1 | 26-05-2005 | NONE | | |
| US 2003087796 | A1 | 08-05-2003 | WO | 0181374 A2 | 01-11-2001 |
| | | | DE | 10021579 A1 | 08-11-2001 |
| | | | EP | 1276456 A2 | 22-01-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20050112684 A1
- US 2007069889 A1
- US 2007069901 A
- EP 291194 B1
- EP 186799 B1

- WO 2005121800 A2
- US 4956302 A
- US 6372513 B
- US 5770460 A
- US 4943522 A

### Non-patent literature cited in the description

- **Wedekind, C. ; D. Penn.** MHC genes, body odours, and odour preferences. *Nephrology Dialysis Transplantation,* 2000, vol. 15, 1269-1271
- **Eggert, F. et al.** The major histocompatibility complex and the chemosensory signalling of individuality in humans. *Genetica,* 1999, vol. 104, 265-273
- **Penn, D.J. ; W.K. Potts.** The evolution of mating preferences and major histocompatibility complex. *The American Naturalist,* 1999, vol. 153 (2), 145-164
- **Wedekind, C. et al.** MHC-dependent mate preferences in humans. *Proc. R. Soc. Lond. B,* 1995, vol. 260, 245-249
- **Singh, P.B.** Chemosensation and genetic individuality. *Reproduction,* 2001, vol. 121, 529-539
- **Penn, D.J. et al.** Individual and gender fingerprints in human body odour. *J. R. Soc. Interface,* 2006, vol. 4, 331-340
- **Restrepo, D. et al.** Odortypes and MHC peptides: complementary chemosignals of MHC haplotype. *Trends in Neurosciences,* 2006, vol. 29 (11
- **Milinski, M. et al.** Mate choice decisions of stickleback females predictably modified by MHC peptide ligands. *Prodeedings of the National Academy of Science,* 2005, vol. 102 (12), 4414-4418
- **Ables, E.M. ; L.M. Kay ; J.M. Mateo.** Rats assess degree of relatedness from human odors. *Physiology & Behavior,* 2007, vol. 90, 726-732
- **Wyart, C. et al.** Smelling a single component of male sweat alters levels of cortisol in women. *Journal of Neuroscience,* 2007, vol. 27 (6), 1261-1265
- **Milinski, M. ; C. Wedekind.** Evidence for MHC-correlated perfume preferences in humans. *Behavioral Ecology,* 2001, vol. 12 (2), 140-149
- **Jacob, S. et al.** Paternally inherited HLA alleles are associated with women's choice of male odor. *Nature Genetics,* 2002, vol. 30 (2), 175-179

- **Garver-Apgar, C.E. et al.** Major histocompatibility complex alleles, sexual responsivity, and unfaithfulness in romantic couples. *PsychSc,* 2006, vol. 17 (10), 830-836
- **Milinski, M.** The major histocompatibility complex, sexual selection, and mate choice. *Annual Review in Ecology, Evolution, and Systematics,* 2006, vol. 37, 159-186
- **Nowak, M.A. ; K. Tarczy-Hornoch ; J.M. Austy.** *Proc. Natl. Acad. Sci.,* 1992, vol. 89, 10896
- **DeBoer, R.J. ; A.S. Perelson.** *Proceedings of the Royal Society London B,* vol. 252, 171
- **Reusch, T.B.H. et al.** Female sticklebacks count alleles in a strategy of sexual selection explaining MHC polymorphism. *Nature,* 2001, vol. 414, 300-302
- **Wegner, K.M. et al.** Parasite selection for immunogenetic optimality. *Science,* 2003, vol. 301, 1343
- **Zerefos, P.G. et al.** Characterization of the human urine proteome by preparative electrophoresis in combination with 2-DE. *Proteomics,* 2006, vol. 6, 4346-4355
- **Singer, A.G. ; G.K. Beauchamp ; K. Yamazaki.** Volatile signals of the major histocompatibility complex in male mouse urine. *Proc. Natl. Acad. Sci.,* 1997, vol. 94, 2210-2214
- **Krieger, J. et al.** Selective activation of G protein subtypes in the vomeronasal organ upon stimulation wiht urine-derived compounds. *The Journal of Biological Chemistry,* 1999, vol. 274 (8), 4655-4662
- **Leinders-Zufall, T. et al.** MHC class / peptides as chemosensory signals in the vomeronasal organ. *Science,* 2004, vol. 306 (5), 1033-1037
- **Montag, S. et al.** "Electronic nose" detects major histocompatibility complex-dependent prerenal and postrenal odor components. *Proc. Natl. Acad. Sci.,* 2001, vol. 98, 9249-9254

- **Boehm, T. ; F. Zufall.** MHC peptides and the sensory evaluation of genotype. *Trends in Neurosciences,* 2006, vol. 29 (2
- **Rammensee, H.-G. et al.** SYFPEITHI: database for MHC ligands and peptide motifs. *Immunogenetics,* 1999, vol. 50, 213-219
- **Yewdell, J.W. ; J.R. Bennink.** Immunodominance in major histocompatibility complex class I-restircet T lymphocyte responses. *Annual Review in Immunology,* 1999, vol. 17, 1-30
- **Zavazava, N. ; G. Leimenstoll ; W. Muller-Ruchholtz.** Measurement of soluble MHC class / molecules in renal graft patients: a noninvasive allograft monitor. *Journal of Clinical Laboratory Analysis,* 1990, vol. 4, 426-429
- **Adamashvili, I. et al.** Soluble HLA: patterns of expression in normal subjects, autoimmune diseases, and transplant recipients. *Rheumatol Int,* 2005, vol. 25, 491-500
- **Wobst, B. et al.** Molecular forms of soluble HLA in body fluids: potential determinants of body odor cues. *Genetica,* 1999, vol. 104, 275-283
- **Adamashvili, I. et al.** Soluble HLA in saliva of patients with autoimmune rheumatic diseases. *Rheumatol Int,* 2002, vol. 22, 71-76
- **Hayman, M.W. et al.** Soluble human leukodyte antigen: A diagnostic indicator of rheumatoid arthritis. *Journal of Immunological Methods,* 2006, vol. 315, 19-26
- **Amirghofran, Z. et al.** *Journal of Cancer Research Clinical Oncology,* 2002, vol. 128, 443-448
- **Utsumi, M. et al.** Expression of major urinary protein genes in the nasal glands associated with general olfaction. *Neurobiology,* 1999, vol. 39, 227-236
- **Brennan, P.A. ; F. Zufall.** Pheromonal communication in vertebrates. *Nature,* 2006, vol. 444, 308
- **Fan, J. et al.** Visual perception of male body attractiveness. *Proceedings of the Royal Society London B,* 2005, vol. 272, 219-226
- **Tovée, M.J. et al.** Effects of sexual dimorphism on facial attractiveness. *Lancet,* 1999, vol. 399, 215-216
- **Perrett, D.I. et al.** Effects of sexual dimorphism on facial attractiveness. *Nature,* 1998, vol. 394, 884-887
- The evolution of desire: strategies of human mating. **Buss, D.M.** Basic Books. 1994
- **Gourab Goshal, P.H.** Attractiveness and activity in internet comunities. *Physica,* 2006, vol. 364, 603-609
- **Schreuder, G.M.T. et al.** The HLA Dictionary 2004: a summary of HLA-A, -B, -C, -DRB 1/3/4/5 and -DQB1 alleles and their association with serologically defined HLA-A, -B, -C, -DR and -DQ antigens. *Tissue Antigens,* 2005, vol. 65, 1-55
- **Singh, P.B. ; R.E. Brown ; B. Roser.** MHC antigens in urine as olfactory recognition cues. *Nature,* 1987, vol. 327, 161-164
- **Reynolds, J.B. ; B.S. Weir ; C.C. Cockerham.** Estimation of the coancestry coefficient: basis for a short-term genetic distance. *Genetics,* 1983, vol. 105, 767-779
- **Cavalli-Sforza, L.L. ; A.W.F. Edwards.** Phyligenetic analysis: models and estimation procedures. *Evolution,* 1967, vol. 32, 550-570
- **Nei, M.** Genetic distance between populations. *American Naturalist,* 1972, vol. 106, 283-292
- **Midleton, D. et al.** New allele frequency database. *Tissue Antigens,* 2003, vol. 61, 403-407, www. allelefrequencies. net